# EUROPÄISCHE PATENTSCHRIFT

(11) **EP 1 201 264 B1**
(45) Veröffentlichungstag und Bekanntmachung des Hinweises auf die Patenterteilung: **29.11.2006**
(21) Anmeldenummer: 01125165.9
(22) Anmeldetag: 23.10.2001
(51) Int. Cl.: A61M 39/22, A61M 1/36

(54) **Wegwerfbare Kassette mit Dichtungsmembran**
Diposable cassette comprising a selaing membrane
Cassette jetable ä membrane

(30) Priorität: 27.10.2000 DE 10053441
(43) Veröffentlichungstag der Anmeldung: 02.05.2002
(73) Patentinhaber: Fresenius Medical Care Deutschland GmbH, 61352 Bad Homburg v.d.H. (DE)
(72) Erfinder: Lauer, Martin, 66606 St. Wendel (DE)
(74) Vertreter: Laufhütte, Dieter

(56) Entgegenhaltungen:
- DE-C- 19 718 963
- US-A- 5 062 774
- US-A- 5 098 262
- US-A- 5 203 368
- US-A- 5 722 946
- US-A- 5 803 105

## Beschreibung

Die vorliegende Erfindung betrifft eine Vorrichtung zur Zuführung und/oder Abzweigung einer Nebenströmung in eine und/oder aus einer Hauptströmung einer medizinischen Flüssigkeit, mit einem Fluidführungskorpus, in dem zumindest ein zu einer Seite offener Hauptkanal sowie zumindest ein Nebenkanal mit einer mit dem Hauptkanal strömungsverbindbaren Mündung vorgesehen sind, und einer Abdeckfolie, die auf dem Fluidführungskorpus über der offenen Seite des Hauptkanals angeordnet ist, wobei die Mündung des Nebenkanals in den Hauptkanal von der Abdeckfolie verschließbar ist.

Es sind Einmalkassetten, sogenannte Kassettendisposables der Membranbauart z.B. aus der WO 97/09074 bekannt, bei denen medizinische Flüssigkeiten wie z.B. Dialysierflüssigkeit in nach einer Seite offenen Kanälen strömt. Die Kanalwände und die zwischen den Kanälen liegenden Flächen werden von einem in der Regel im Spritzguß ausgeführten Kassettenkorpus gebildet. Die nach einer Seite offenen Kanäle werden durch eine aufgelegte Abdeckfolie abgedichtet, z.B. durch Anschweißen entlang der Kanalränder oder durch Aufpressen, ggf. auf erhöht ausgeführte Kanalränder.

Eine Maschine, in die ein solches Kassettendisposable eingelegt wird, enthält in der Regel die Aktorik für Mess-, Pump- und Ventilfunktionen und klemmt die Kassette mit der daraufliegenden Abdeckfolie flächig ein. Die auf der Abdeckfolie aufliegende maschinenseitige Fläche besteht in ihrer Randschicht in der Regel aus einem Elastomer.

Aus DE 197 18 963 C1 ist ein Steuerventil gemäß dem Oberbegriff des vorliegenden Anspruchs 1 bekannt, bei dem ein Zulauf und ein Ablauf als Nuten ausgeführt sind, welche nebeneinander verlaufen und durch einen Steg voneinander getrennt sind. Ein flexibles Sperrglied liegt im geschlossenen Zustand flüssigkeitsdicht auf der Anordung auf. Zum Öffnen des Steuerventils wird das Sperrglied angehoben, so dass oberhalb des Stegs eine Verbindung zwischendem Zulauf mit dem Ablauf entsteht.

Insbesondere bei Kassettendisposables, die Teil eines extrakorporalen Blutkreislaufes zur extrakorporalen Blutbehandlung sind, besteht in verfahrenstechnischer Hinsicht das Erfordernis, dass in diese Kanäle wahlweise Flüssigkeiten wie Infusionslösungen, Medikamente, Heparin, Substituat und dergleichen hineindosiert oder Flüssigkeitsproben aus den Kanälen herausgezogen werden sollen. Hierzu sind Ventilstellen erforderlich, um den Hauptkanal, durch den die medizinische Flüssigkeit strömt, mit einem entsprechenden Nebenkanal zu verbinden. Regelmäßig ist dieser Nebenkanal geschlossen, da das Zudosieren bzw. die Probenahmen nur kurze Zeit beanspruchen.

Derartige Vorrichtungen unterliegen vielfältigen Anforderungen, die vom Stand der Technik noch nicht zufriedenstellend gelöst sind. Grundsätzlich sollen die zu erfüllenden Funktionen mit einer kompakten Anordnung erreicht werden. Bei einer Hineinverlegung des Nebenkanals in den Hauptkanal ergeben sich jedoch Probleme bezüglich einer turbulenz- und widerstandsarmen Strömung der medizinischen Flüssigkeit in dem Hauptkanal, insbesondere wenn es sich dabei um Blut handelt.

Darüber hinaus neigt die Abdeckfolie bei bekannten Anordnungen zur Ermüdung in Folge des Geschlossenhaltens des Nebenkanals über lange Zeit.

Der vorliegenden Erfindung liegt daher die Aufgabe zugrunde, eine verbesserte Vorrichtung der eingangs genannten Art zu schaffen, die die Nachteile des Standes der Technik vermeidet und diesen in vorteilhafter Weise weiterbildet. Vorzugsweise soll mit einer einfachen, kompakten und kostengünstigen Anordnung das Zudosieren bzw. die Probennahmen schließsicher und verschleißarm ermöglicht werden, ohne die Strömungsverhältnisse der medizinischen Flüssigkeit nachhaltig zu stören.

Diese Aufgabe wird erfindungsgemäß durch eine Vorrichtung nach Patentanspruch 1 gelöst. Vorteilhafte Ausgestaltungen der Erfindung sind Gegenstand der Unteransprüche.

Dabei sind der Hauptkanal und der Nebenkanal voneinander lediglich durch einen Steg getrennt, der eine Seitenwandung des Hauptkanals bildet und diese zu den angrenzenden Bereichen hin stetig fortsetzt. Der Nebenkanal erstreckt sich dabei senkrecht auf den Hauptkanal hin zu, wobei sich der Querschnitt des Nebenkanals zu dem trennenden Steg hin deltaförmig erweitert.

Das Anheben der elastomeren Abdeckfolie (wie auch evtl. der elastomeren Maschinenstempel-Abdeckmembran) ähnelt dem Öffnen von Lippen: Da Folie und Membran nicht überdehnt werden dürfen, um später faltenfrei wieder schließen zu können, ist der Öffnungshub relativ klein und die Öffnungsbreite relativ groß. Daher die deltaförmige Erweiterung des normalerweise schmalen Nebenkanals.

Der Nebenkanal, dessen Mündung und der Hauptkanal sind also derart strömungsgünstig gestaltet, dass sich im Bereich der Mündung des Nebenkanals eine totraumfreie Hauptkanalströmung einstellt, die sich von der Strömung in den ventilfreien Abschnitten des Hauptkanals nicht charakteristisch unterscheidet. Diese Strömungsqualitäten verbessern die Hämokompatibilität der Vorrichtung wesentlich.

Besondere Vorteile ergeben sich bei der Verwendung der obenbeschriebenen Vorrichtung in Verbindung mit Blut, also bei der Zuführung einer medizinischen Flüssigkeit in einen Blutstrom und/oder der Probennahme aus einem Blutstrom. Die Stromlinienform des Nebenkanals verhindert ein Stehen des Blutstroms in Toträumen oder Strömungsschatten hinter dem Nebenkanal sowie eine blutschädigende Turbulenzbildung.

Die erfindungsgemäße Vorrichtung zur Zuführung und/oder Abzweigung einer Nebenströmung in eine und/oder aus einer Hauptströmung findet besonders vorteilhaft Anwendung als Teil eines extrakorporalen Blutkreislaufs zur extrakorporalen Blutbehandlung. Eine solche Blutbehandlung kann beispielsweise die Hämodialyse, die Hämofiltration, die Blutzellseparation oder die Blutadsorption umfassen. Bei dieser Anwendung ist die Vorrichtung vorteilhafterweise als Disposable für den Einmalgebrauch ausgebildet. Der Ventilaktor ist in diesem Fall zumeist ein Teil der Blutbehandlungsmaschine, die die Flüsse der beteiligten Fluide steuert.

Nachfolgend wird die Erfindung anhand einer bevorzugten Ausführungsform und zugehörigen Zeichnungen näher erläutert. In den Zeichnungen zeigt:

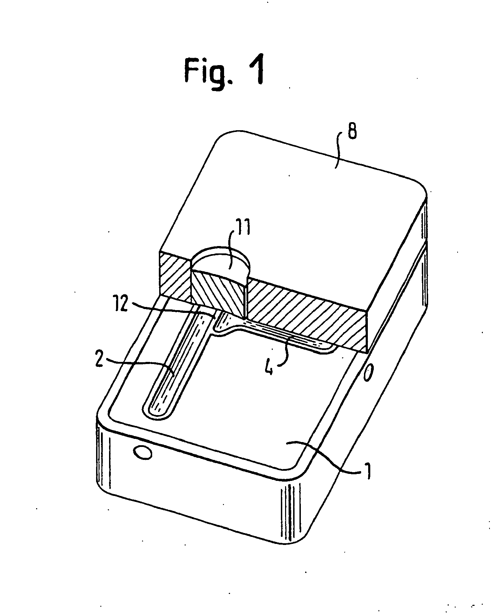

- Fig. 1:: eine perspektivische Ansicht eines Fluidführungskorpus mit einem offenen Hauptkanal und einem in diesen mündenden Nebenkanal gemäß einer weiteren bevorzugten Ausführung der Erfindung.

Der in der Figur gezeigte Fluidführungskorpus mit darauf liegender Abdeckfolie kann grundsätzlich als Disposablebauteil zum Einfügen in Schlauchsysteme oder als permanent genutztes Membranventil in der chemisch-pharmazeutischen Verfahrenstechnik Verwendung finden. Insbesondere jedoch ist der dargestellte Fluidführungskorpus mit der zugehörigen Abdeckfolie ein Kassettendisposable vom Membrantyp für medizinische Flüssigkeiten, vorzugsweise für Blut für die extrakorporale Behandlung des Blutes wie z. B. der Hämodialyse.

Eine Ausführung der Erfindung zeigt Figur 1. Der Nebenkanal 4 mündet bei dieser Ausführung nicht in Form eines vulkanartigen Schlotes in den Hauptkanal 2. Vielmehr sind der Hauptkanal 2 und der Nebenkanal 4, die beide, der Nebenkanal 4 zumindest teilweise, in dem Fluidführungskorpus 1 als offene Nut ausgebildet sind, voneinander lediglich durch einen Steg 12 getrennt, der eine Seitenwandung des Hauptkanals 2 bildet und diese zu den angrenzenden Bereichen hin stetig fortsetzt (vgl. Figur 1). Der Nebenkanal erstreckt sich bei der gezeigten Ausführung senkrecht auf den Hauptkanal 2 hin zu, wobei sich der Querschnitt des Nebenkanals 4 zu dem trennenden Steg 12 hin deltaförmig erweitert.

Das Anheben der elastomeren Abdeckfolie (wie auch evtl. der elastomeren Maschinenstempel-Abdeckmembran) ähnelt dem Öffnen von Lippen: Da Folie und Membran nicht überdehnt werden dürfen, um später faltenfrei wieder schließen zu können, ist der Öffnungshub relativ klein und die Öffnungsbreite relativ groß. Daher die deltaförmige Erweiterung des normalerweise schmalen Nebenkanals.

Die Verbindung des Nebenkanals 4 mit dem Hauptkanal 2 wird durch die auf dem Fluidführungskorpus 1 aufliegende Abdeckfolie 3, die die offenen Seiten des Nebenkanals 4 und des Hauptkanals 2 überspannt, gesteuert. Die Abdeckfolie ist in Figur 1 nicht dargestellt. Die Abdeckfolie kann um den Hauptkanal 2 unter Aussparung des Trennsteges 12 sowie um den Nebenkanal 4 herum auf den Fluidführungskorpus 1 aufgeschweißt sein. Die Abdeckfolie kann jedoch auch von einem Ventilstempel, der von einem Maschinenblock gebildet sein kann, gegen die Oberfläche des Fluidführungskorpus 1 gedrückt werden. Um den Nebenkanal 4 mit dem Hauptkanal 2 in Strömungsverbindung zu bringen, ist ein Ventilaktor bzw. der Betätigungsteil 11 vorgesehen, der im Bereich des Steges 12 auf dem Fluidführungskorpus 1 sitzt und die Abdeckfolie auf den Steg 12 drückt. Wird der Betätigungsteil 11 von dem Fluidführungskorpus 1 weggefahren, kann die Folie von dem Steg 12 wegbewegt werden, so daß über diesen hinweg der Hauptkanal 2 mit
dem Nebenkanal 4 in Strömungsverbindung gebracht wird. Es versteht sich, dass in der Figur 1 der Maschinenblock 11 geschnitten dargestellt ist und sich über die gesamte Fläche des Fluidführungskorpus 1, in der die Kanäle offen zu Tage treten, erstreckt.

An den Lippenrändern grenzen stillstehene und bewegliche Dichtzonen unmittelbar aneinander an.

Bei dieser Ausführungsform bleibt die Hauptkanalströmung von dem Nebenkanal 4 bzw. dessen Mündung gänzlich unbeeinflusst. In geschlossener Stellung, d. h. wenn das Betätigungsteil 11 auf den Fluidführungskorpus 1 gedrückt ist, ist aus der Sicht des durchströmten Hauptkanals kein Ventil bzw. kein Nebenkanal vorhanden, so dass optimale Strömungsverhältnisse erreicht werden können.

## Patentansprüche

1. Vorrichtung zur Zuführung und/oder Abzweigung einer Nebenströmung in eine und/oder aus einer Hauptströmung einer medizinischen Flüssigkeit, mit
a.) einem Fluidführungskorpus (1), in dem zumindest ein zu einer Seite offener Hauptkanal (2) sowie zumindest ein Nebenkanal (4), der eine mit dem Hauptkanal (2) in Strömungsverbindung bringbare Mündung (7) besitzt, vorgesehen sind, und
b.) einer Abdeckfolie (3), die auf dem Fluidführungskorpus über der offenen Seite des Hauptkanals angeordnet ist,
c.) wobei die Mündung (7) des Nebenkanals (4) von der Abdeckfolie (3) verschließbar ist, und
d.) wobei der Nebenkanal (4) derart ausgebildet ist, dass der Hauptkanal (2) im Bereich der Mündung (7) des Nebenkanals (4) im wesentlichen totraumfrei ist,
e.) wobei Hauptkanal (2) und Nebenkanal (4) lediglich durch einen Steg (12) voneinander getrennt sind, der eine Seitenwandung des Hauptkanals bildet und diese zu den angrenzenden Bereichen hin stetig fortsetzt, und
f.) wobei der Nebenkanal (4) zumindest teilweise in dem Fluidführungskorpus (1) als offene Nut ausgebildet ist
**dadurch gekennzeichnet,**
**dass** der Nebenkanal (4) mit seiner Längsachse senkrecht auf die Strömungsrichtung der Hauptströmung mündet und dass sich der Querschnitt des Nebenkanals (4) zu dem Steg (12) hin deltaförmig erweitert.

2. Vorrichtung nach Anspruch 1, wobei die Abdeckfolie (3) aus Kunststoff besteht.

3. Vorrichtung nach Anspruch 2, wobei die Abdeckfolie (3) von einer elastischen Membran gebildet wird.

4. Vorrichtung nach einem der vorhergehenden Ansprüche, wobei der Fluidführungskorpus (1) mit der Abdeckfolie (3) eine Disposablekassette bildet, die in eine Maschine zur extrakorporalen Blutbehandlung eingesetzt ist.

5. Vorrichtung nach Anspruch 4, wobei die Maschine zur extrakorporalen Blutbehandlung eine Dialysemaschine ist.

## Claims

1. Apparatus for supplying and/or branching off a secondary flow into and/or out of a main flow of a medical fluid, comprising
a) a fluid guide body (1) in which there are provided at least one main passage (2) open to one side and at least one secondary passage (4) having a mouth opening (7) which can be brought into flow communication with the main passage (2), and
b) a cover film (3) arranged on the fluid guide body over the open side of the main passage,
c) wherein the mouth opening (7) of the secondary passage (4) is closable by the cover film (3) and
d) wherein the secondary passage (4) is so formed that the main passage (2) is substantially free of dead space in the region of the mouth opening (7) of the secondary passage (4),
e) wherein the main passage (2) and the secondary passage (4) are only separated from each other by a limb (12) which forms a side wall of the main passage and continuously extends same towards the adjoining regions and
f) wherein the secondary passage (4) is at least partially in the form of an open groove in the fluid guide body (1),
**characterised in that**
the secondary passage (4) opens with its longitudinal axis perpendicularly to the flow direction of the main flow and that the cross-section of the secondary passage (4) enlarges in a delta shape towards the limb (12).

2. Apparatus according to claim 1 wherein the cover film (3) comprises plastic material.

3. Apparatus according to claim 2 wherein the cover film (3) is formed by an elastic membrane.

4. Apparatus according to one of the preceding claims wherein the fluid guide body (1) with the cover film (3) forms a disposable cassette which is fitted into a machine for extra-corporeal blood treatment.

5. Apparatus according to claim 4 wherein the machine for extra-corporeal blood treatment is a dialysis machine.

## Revendications

1. Dispositif pour l'amenée et/ou le piquage d'un écoulement secondaire dans un et/ou depuis un écoulement principal d'un liquide médical, avec
a.) un corps de guidage du fluide (1) dans lequel sont prévus au moins un canal principal (2) ouvert sur un côté, ainsi qu'au moins un canal secondaire (4), qui possède une embouchure (7) pouvant être amenée en communication d'écoulement avec le canal principal (2), et
b.) une feuille de recouvrement (3) qui est disposée sur le corps de guidage du fluide au-dessus de la face ouverte du canal principal,
c.) sachant que l'embouchure (7) du canal secondaire (4) peut être fermée par la feuille de recouvrement (3) et
d.) sachant que le canal secondaire (4)est configuré de telle sorte que, dans la zone de l'embouchure (7) du canal secondaire (4), le canal principal (2) est essentiellement exempt de volume mort,
e.) sachant que le canal principal (2) et le canal secondaire (4) ne sont séparés l'un de l'autre que par une arête (12), qui forme une paroi latérale du canal principal et prolonge celle-ci continûment vers les zones contiguës, et
f.) sachant que le canal secondaire (4) est configuré au moins en partie dans le corps de guidage du fluide (1) comme rainure ouverte,
**caractérisé en ce que**
le canal secondaire (4) débouche, avec son axe longitudinal, perpendiculairement à la direction de l'écoulement de l'écoulement principal et **en ce que** la section du canal secondaire (4) s'élargit vers l'arête (12) en forme de delta.

2. Dispositif selon la revendication 1, dans lequel la feuille de recouvrement (3) est en plastique.

3. Dispositif selon la revendication 2, dans lequel la feuille de recouvrement (3) est formée d'une membrane élastique.

4. Dispositif selon l'une des revendications précédentes, dans lequel le corps de guidage du fluide (1) forme avec la feuille de recouvrement (3) une cassette jetable qui est utilisée dans une machine pour le traitement extracorporel du sang.

5. Dispositif selon la revendication 4, dans lequel la machine pour le traitement extracorporel du sang est un dialyseur.
